# EUROPEAN PATENT APPLICATION

(11) **EP 3 040 723 A1**
(43) Date of publication of application: **06.07.2016**
(21) Application number: 14840291.0
(22) Date of filing: 31.07.2014
(51) Int. Cl.: G01N 33/53, B23K 20/10, G01N 37/00

(54) **BIOCHIP SUPPORT MEMBER, METHOD FOR MANUFACTURING BIOCHIP SUPPORT MEMBER, BIOCHIP PACKAGE, SCREENING DEVICE, AND SCREENING METHOD**

(30) Priority: 30.08.2013 JP 2013180248
(71) Applicant: Nikon Corporation, Tokyo 108-6290 (JP)
(72) Inventor: ISAMI, Tadao, Tokyo 100-8331 (JP); HAYASHI, Yutaka, Tokyo 100-8331 (JP); UEDA, Takehiko, Tokyo 100-8331 (JP)
(74) Representative: Hoffmann Eitle
(86) International application number: PCT/JP2014/070230
(87) International publication number: WO 2015/029691

(57) **Abstract**

A biochip support member is provided that comprises a base material, and a columnar member different member from the base material having a support area that can support a biochip formed of a biomolecule provided at one end, and is attached to the base material through an attaching part at the other end.

## Description

### (Technical Field)

The present invention relates to a biochip support member, a method for manufacturing the biochip support member, a biochip package, a screening device, and a screening method.

### (Background Art)

In recent years, by using a microarray (a biochip), where a biomolecule (a probe) is fixed in a substrate, technologies for detecting and diagnosing specific reactions occurring when specimens (target substances) are bound have advanced in the fields including new drug development processing and medical diagnosing. An array plate that uses an adhesive and the like to bond a plate that provides a microarray and a plate that forms a well is disclosed in Patent Document 1.

### (Citation List)

### (Patent Literatures)

Patent Literature 1: Japanese Unexamined Patent Application Publication (Translation of PCT Application) No. 2005-513457

### (Summary of Invention)

### (Techincal Problem)

However, the prior art described above has the following technological problems.

While it is preferable to bring a detector and a biochip as close together as possible when performing fluorescence detection of a micro biochip with a high degree of accuracy, placing a sample and the detector close together has some limitation because the biochip is arranged in the bottom of a well part.

A purpose of the present invention is to provide a biochip support member, a method for manufacturing the biochip, a biochip package, a screening device, and a screening method that makes the biochips simple and easy to measure.

### (Solution to Problem)

According to a first mode of the present invention, a biochip support member is provided that comprises a base material, and a columnar member different from the base material, having a support area that can support a biochip formed of a biomolecule provided at one end, attached to the base material through an attaching part at the other end.

According to a second mode of the present invention, a biochip support member is provided that comprises a base material, a columnar member different from the base material, having a support area that can support a biochip formed of a biomolecule provided at one end, and a welded part interposed between the base material and the other end of the columnar member.

According to a third mode of the present invention, a biochip package is provided that comprises the biochip support member of the first mode or the second mode of the present invention, and a holding member that has a holding part that is able to hold a specimen containing a target that is able to react specifically to a biomolecule.

According to a fourth mode of the present invention, a screening device is provided that comprises a dispenser for dispensing a specimen into the holding part of the biochip package of the third mode of the present invention, and a detector for detecting the affinity between a target contained in the specimen and a biomolecule.

According to a fifth mode of the present invention, a method for manufacturing a biochip support member is provided that comprises a first step that prepares a base material, a second step that prepares a columnar member different from the base material, having a support area that can support a biochip formed of a biomolecule provided at one end, and a third step that attaches the other end of the columnar member to the base material through an attaching part.

According to a sixth mode of the present invention, a screening method is provided that comprises preparing a biochip support member manufactured by the method for manufacturing a biochip support member of the fifth mode of the present invention, preparing a holding part that is able to hold a specimen containing a target that is able to react specifically to a biomolecule, arranging and fixing a biochip in a support area of a columnar member, dispensing the specimen in the holding part so that the biochip is soaked in the specimen, causing a reaction between the target and the biomolecule in the holding part into which the specimen was dispensed, separating the biochip support member and holding member after the reaction, and detecting the affinity between the target and the biomolecule.

### (Effect of the Invention)

With the present invention, it is possible to measure a biochip simply and easily.

### (Brief Description of Drawings)

FIG. 1 is an external perspective view of a biochip support member CP according to an embodiment of the present invention.
FIG. 2 is a biochip support member CP according to an embodiment of the present invention, where (a) is a plan view viewed from a columnar member 10 side, (b) is a visualized cross sectional view of line A-A in (a), (c) is a visualized cross sectional view of line B-B in (a), and (d) is a plan view viewed from a base material BP side.
FIG. 3 is a descriptive view of ultrasonic welding according to an embodiment of the present invention.
FIG. 4 is a descriptive view of ultrasonic welding according to an embodiment of the present invention.
FIG. 5 is a flow chart illustrating a method for manufacturing a biochip support member according to a first embodiment of the present invention.
FIG 6 is a view illustrating the procedure of a method for manufacturing a biochip support member according to a second embodiment of the present invention.
FIG. 7 is a view illustrating the procedure of a method for manufacturing the biochip support member according to the second embodiment of the present invention.
FIG. 8 is a view illustrating the procedure of a method for manufacturing a biochip support member according to a third embodiment of the present invention.
FIG. 9 is a view illustrating the procedure of a method for manufacturing the biochip support member according to the third embodiment invention.
FIG. 10 is an external perspective view of a biochip support member according to an embodiment of the present invention where the biochip has been fixed.
FIG. 11 is a plan view of a well plate according to an embodiment of the present invention.
FIG 12 is a cross sectional view of a biochip package according to an embodiment of the present invention.
FIG 13 is a schematic view of a screening device SC according to an embodiment of the present invention.
FIG. 14 is a schematic configuration drawing of a measuring device according to an embodiment of the present invention.
FIG. 15 is a flow chart illustrating a procedure of a screening method according to an embodiment of the present invention.
FIG. 16 is a plan view illustrating a biochip support member with a different form.
FIG. 17 is a plan view illustrating a columnar member 10 and a holding part 25 having different forms.

### (Description of Embodiments)

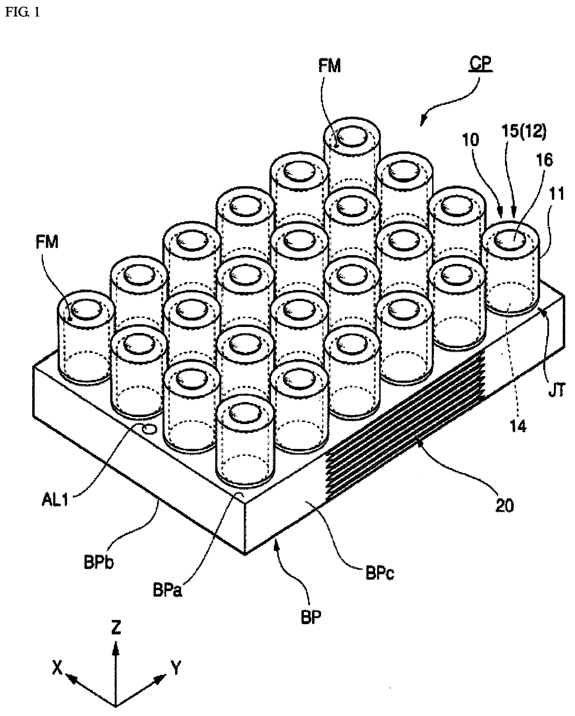

Embodiments of the biochip support member, the method for manufacturing a biochip support member, the biochip package, the screening device, and the screening method of the present invention are described hereinafter with reference to FIG. 1 through FIG. 17.

Note that in the description that follows, the vertical direction is described as the Z axis direction, the long direction (the direction where a columnar member 10 is aligned in six columns) of a biochip support member BP in the horizontal direction is described as the Y axis direction, and the direction orthogonal to the Z axis direction and the Y axis direction is described as the X axis direction. The X axis, Y axis and Z axis are intersecting coordinates.

### [Biochip Support Member]

The biochip support member will be described first with reference to FIG 1 through FIG. 4.

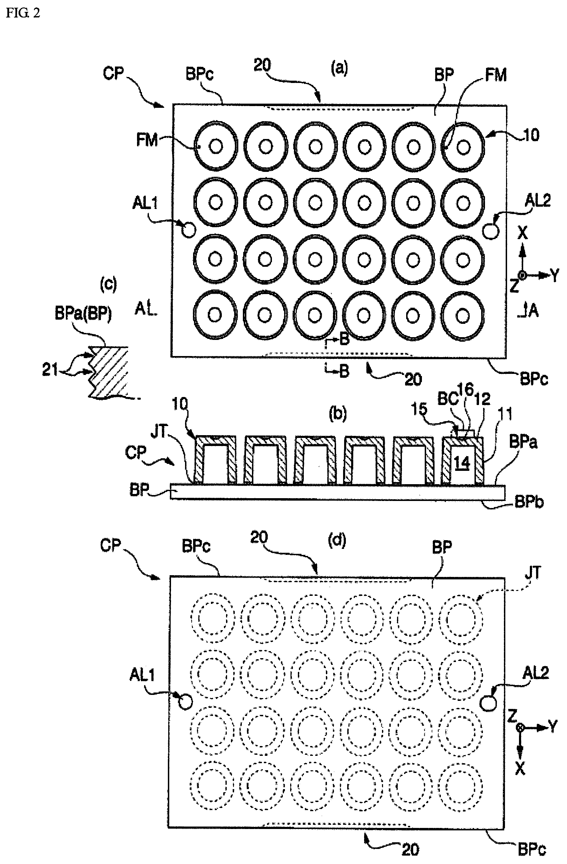

FIG 1 is an external perspective view of a biochip support member CP. FIG 2(a) is a plan view of the biochip support member CP seen from the columnar member 10 side. FIG 2(b) is a visualized cross sectional view of line A-A in FIG 2(a). FIG 2(c) is a visualized cross sectional view of line B-B in FIG. 2(a). FIG. 2(d) is a plan view of the biochip support member CP seen from a base material BP side.

The biochip support member CP provides a base material BP, a columnar member 10 and an attaching part JT. The base material BP is, for example, a plate member with a flat rectangular shape. The base material BP is configured of, for example, a resin. As an example, the base material BP is configured of a thermoplastic resin. The base material BP is manufactured using, for example, a casting method. The base material BP is, for example, a translucent member. As an example, the base material BP is a transparent member. Examples of resins used in the base material BP include acrylic resins such as poly methyl methacrylate resins, polycarbonate (PC), cycloolefm copolymers (COC), and polystyrene (PS).

A plurality of columnar members 10 are aligned in a matrix on a front surface (first surface) BPa of the base material BP. As an example, the plurality of columnar members 10 are aligned along the X direction and the Y direction in a matrix on the front surface (the first surface) BPa of the base material BP. Twenty four pieces of the columnar members 10 are aligned on the front surface BPa of the base material BP, configuring six columns in the longer direction, and four rows in the shorter direction of the base material BP. The front surface BPa and a rear surface (second surface) BPb of the base material BP are finished with a lapping treatment (mirror finish).

A first hole part (hole) AL1 and a second hole part (elongated hole) AL2 are formed, one on either side of the base material BP, in the Y direction as positioning parts for positioning the X and Y directions and θZ direction of the base material on a stage. The first hole part AL1 and the second hole part AL2 are formed so as to have different shapes one from the other, and so as to have predetermined positional relationships with the plurality of columnar members 10. The first hole part AL1 is formed as a through hole that is circular when viewed in a plane. For example, the second hole part AL2 is shaped differently from the first hole part AL1, and is formed as a through hole that is an elongated circle (for example, an ellipse), when viewed in a plane, that extends in the Y direction (or the X direction). A straight line that passes through the center of the first hole part AL1 and the center of the second hold part AL2 is parallel to the Y direction. The shorter diameter side of the ellipse of the second hole part AL2 is formed to the same diameter as the first hole part AL1.

The base material BP provides a gripping part 20 formed on each side surface (third surfaces) BPc of the longer sides formed along the outer edge of the front surface BPa and the outer edge of rear surface BPb. As illustrated in FIG. 2(c), the gripping part 20 provides a plurality of grooves 21 that extend in the Y direction and are formed so that the bottoms thereof have V shaped cross sections. A plurality of the grooves 21 are formed along the Z direction. The gripping part 20 is provided in a range so as to be symmetrical from the center of the Y direction on each side surface BPc. The length of the gripping part 20 is set to be, for example, 1/2 or more of the length of the side surface BPc.

The columnar member 10 is attached to the base material BP through the attaching part JT. The attaching part JT is formed on a portion of the columnar member 10. The columnar member 10 is attached to the base material BP by melting the attaching part JT by welding. When the base material BP is configured of a translucent member, a welded part (welded layer) occurring due to the fact that the attaching part JT is melted by welding, can be observed from the rear surface BPb side of the base material, as illustrated in FIG. 2(d). The shape of the welded part (welded layer) appears in a shape that corresponds to the shape of the attaching part JT (for example, a protrusion 13 that will be described later) before being melted by welding.

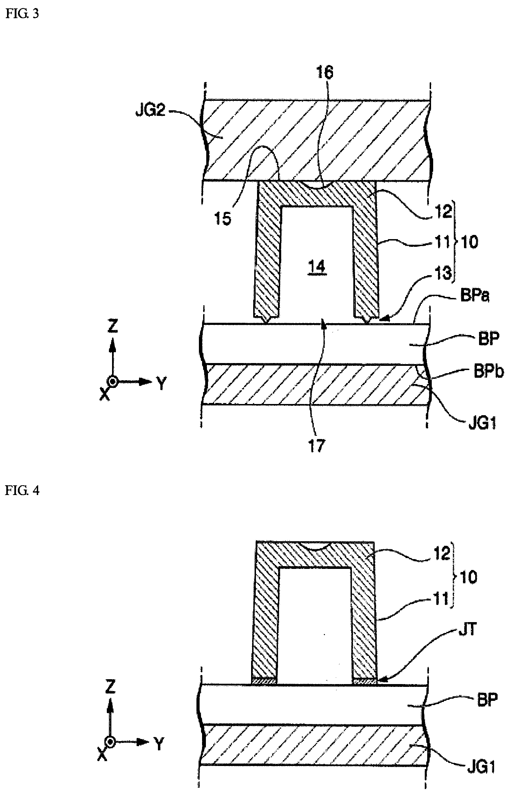

FIG. 3 illustrates the columnar member 10, a holding tool JG1, and an ultrasonic horn JG2. It should be noticed that FIG. 3 illustrates the columnar member 10 before being attached (before welding) to the base material BP by welding. The columnar member 10 before welding, provides a cylindrical part 11, a top panel 12 provided on one end (the + Z side in FIG. 2(b) and FIG. 3) of the cylindrical part 11, and a protrusion 13 provided on the other end (- Z side in FIG. 3) of the cylindrical part 11.

The cylindrical part 11 provides an outer surface where the diameter gradually increases from one end to the other, and an inner surface where the diameter gradually increases going from one end to the other.

The top panel 12 has a support area 15 that is able to support a biochip CP As illustrated in FIG 2(b), the top panel 12 provides the support area 15 that is able to support a biochip BC on the surface of one end.

The columnar member 10 has a mark FM. The mark FM is an index indicating the position of the columnar member 10 in the base material BP. The mark FM is provided in the support area 15. As an example, the mark FM is provided in an area of the support area 15 that is not the area where the biochip BC is fixed. The mark FM is provided on a straight line that passes through the center of the columnar member 10 and is parallel to the Y direction. The mark FM is provided on the - Y side from the center of the columnar member 10. Of the plurality of columnar members 10 in FIG. 2(a), the FM mark is provided on the columnar member 10 that is positioned the farthest to the +X side on the column that is the farthest to the +Y side, and on the columnar member 10 that is positioned the farthest to the +X side on the column that is the farthest to the -Y side. That is, the biochip support member CP has two marks FM. As an example, the two marks FM are provided on a straight line that is parallel to the Y direction and that passes through the center of the columnar member 10 (support area 15) that is positioned the farthest to the +X side on the column that is the farthest to the +Y side, and through the center of the columnar member 10 (support area 15) that is positioned the farthest to the +X side on the column that is the farthest to the -Y side. The number of marks FM is not limited to this. A plurality of marks FM may be provided on a straight line that is parallel to a straight line that passes through the centers of each of the plurality of columnar members 10 rather than on a straight line that passes through the center of each of the plurality of columnar members 10. The mark FM can appropriately be selected from shapes such as, for example, a round shape, a rectangular shape, an L shape, and a cross shape. The mark FM is provided by, for example, performing processing in the support area 15.

The top panel 12 has a dent 16. The dent 16 is recessed from the support area 15. The dent 16 has a concave shape. The surface of the dent 16 is, for example, spherical. The outer diameter of the dent 16 in the surface of the top panel 12 is set smaller than the outer diameter of the biochip BC. That is, the dent 16 is provided at a size so as to be shielded by the biochip BC and thus is not exposed when the biochip BC is fixed in the support area 15.

Returning to FIG. 3, the protrusion 13 protrudes from an end surface of the cylindrical part 11. The tip of the protrusion 13 has a V shaped cross section that forms an edge contour that is provided in a ring shape (annular shape) at the tip of the cylindrical part 11. Therefore, when a welding process is performed that applies pressure and ultrasonic vibration (to be described in detail later) in a state where the protrusion 13 is caused to abut the front surface BPa of the base material BP, the protrusion 13 melts to become the attaching part (welded part) JT that fixes the columnar member 10 to the base material BP, as illustrated in FIG. 4.

The columnar member 10 is closed on one end by the top panel 12 and has an opening 17 on the other end that is attached to the base material BP. The columnar member 10 has a concave part that is surrounded by the cylindrical part 11 and the top panel 12 and is open on the other end. The columnar member 10 is formed into the shape of a cap. The columnar member 10 has an internal space 14 that is formed by the concave part that is surrounded by the cylindrical part 11 and the top panel 12.

The columnar member 10 is configured of, for example, a resin. As an example, the columnar member 10 is configured of a thermoplastic resin. The columnar member 10 is manufactured by, for example, injection molding. The columnar member 10 is, for example, a translucent member. As an example, the columnar member 10 is a transparent member. Examples of resins used in the columnar member 10 include acrylic resins such as poly methyl methacrylate resins and the like, polycarbonate (PC), cycloolefin copolymers (COC), polystyrene (PS) and the like.

When the columnar member 10 is attached to (joined with) the base material BP by welding, it is preferable that the melting point of the material used in the columnar member 10 be the same as the melting point of the material used in the base material BP and, from this perspective, it is preferable that the columnar member 10 and the base material BP be formed using the same material. Furthermore, it is preferable that the melting point of the material used in the columnar member 10 be close to the melting point of the material used in the base material BP even when different types of materials are used in the columnar member 10 and in the base material BP. As an example of such a case, an example can be given where poly methyl methacrylate is used in one of either the columnar member 10 or the base material BP, and polycarbonate (PC) is used in the other of either the columnar member 10 or the base material BP.

### [Method for Manufacturing the Biochip Support Member (First Embodiment)]

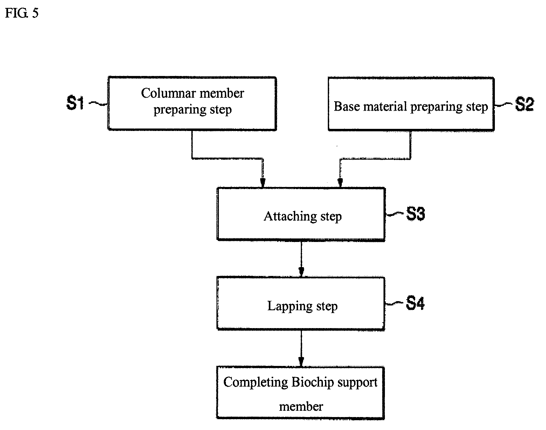

The first embodiment of the method for manufacturing the biochip support member CP described above will be described hereinafter with reference to the flow chart illustrated in FIG. 5.

As illustrated in FIG. 5, the method for manufacturing the biochip support member CP includes a columnar member preparing step (second step) S1, a base material preparing step (first step) S2, an attaching step (third step) S3, and a lapping step (fourth step) S4.

The columnar member 10 prior to welding illustrated in FIG. 3 is prepared in the columnar member preparing step S1. For example, when the columnar member 10 is manufactured by injection molding using a mold, the outside surface of the cylindrical part 11, the support area 15 and the dent 16 are formed using a stationary mold, and the end surface on the other end of the cylindrical part 11, the internal space 14 and the protrusion 13 are formed using a movable mold, setting the end surface position on the other end of the cylindrical part 11 as the parting line (mold opening and closing position). In this case, by providing a gate (molten resin inlet) in the dent 16 in the stationary mold, projections can be prevented from protruding from the support area 15 even if such projections remain on the columnar member 10 from the gate cut marks.

Furthermore, it is preferable, with regard to a columnar member 10 that provides the mark FM, that, for example, a projection corresponding to the mark shape be formed in the mold, and that the mark FM be formed by the injection molding described above.

For example, a base material BP manufactured by a casting method is prepared in the base material preparing step S2. The gripping part 20, first hole part AL1, and second hole part AL2 are formed in the base material BP at the base material preparing step S2. Furthermore, a lapping treatment (for example, diamond lapping) is carried out to a rear surface BPb of the base material BP in the base material preparing step S2.

Once the preparation of both the base material BP and the columnar member 10, which is formed as a separate member (separate body) from the base material BP, is completed, the attaching step S3, which attaches the columnar member 10 to the base material BP, is executed.

As illustrated in FIG 3, a welding process using the holding tool JG1 and an ultrasonic horn (oscillator) JG2 is performed in the attaching step S3. The holding tool JG1 supports the rear surface BPb of the base material BP. The ultrasonic horn JG2 applies ultrasonic vibration and pressure to an object to be welded.

First, the base material BP is mounted on the holding tool JG1 so that the rear surface BPb abuts thereon. Next, the columnar member 10 is mounted on the front surface BPa of the base material BP at a predetermined position. The mounting of the columnar member 10 involves, for example, a transport device (not illustrated in the figure) that is able to hold using negative pressure suction that attaches and holds the support area 15 of the columnar member 10 and then transport the member to the predetermined position on the base material BP. When the welding process is performed relative to the columnar members 10 individually, the ultrasonic horn JG2 is abutted against the supporting areas 15 in succession. When the welding process is performed relative to a plurality (24 in this case) of columnar members 10 all at once, the support areas 15 are abutted against the ultrasonic horn JG2 after all of the columnar members 10 have been mounted on the base material BP.

As for the method for transporting the columnar member 10 to above the base material BP, a method that transports one at a time and a method the transports a plurality all at once can be applied as appropriate. For example, when the welding process is performed in a continuous manner after the injection molding of the columnar member 10, it is possible, with the arrangement of the cavities in the mold being the same as the arrangement of the columnar members 10 in the base material BP, for the transport device to receive and then attach and hold all of the columnar members 10 released from the mold all at once, and then mount all of the held columnar members 10 on the base material BP all at once.

After the columnar member 10 is mounted on the base material BP, the ultrasonic horn JG2 is mounted on the upper part of the columnar member 10, and then the ultrasonic horn JG2 applies ultrasonic vibration and pressure to the columnar member 10. By this, frictional heat is generated between the protrusion 13 of the columnar member 10 and the base material BP, and the protrusion 13, where the stress is primarily prone to concentrate, will melt and then solidify (cure), and will thus fix (join) the columnar member 10 to the base material BP as the attaching part (welded part, welded layer) JT, as illustrated in FIG. 4. At this time, because the opening 17 in the internal space 14 is closed by the base material BP, a closed space is formed inside the columnar member 10. A closed space is formed inside the columnar member 10 by the opening 17, the welded part, and the base material BP.

When the columnar member 10 has been attached to the base material BP through the attaching part JT, the process proceeds to the lapping step S4. With the lapping treated rear surface BPb of the base material BP as a reference surface, a lapping treatment (for example, diamond lapping) is carried out to the support area 15 of the columnar member 10 in the lapping step S4. By this, the support area 15 - which will become a support surface for the biochip BC - is flattened and smoothed, with the rear surface PBb - which will become a support surface during measurement in a subsequent step - as a reference surface. When a plurality of the columnar members 10 are attached to the base material BP, the lapping treatment is performed on all of the columnar members 10. In this way, the columnar member 10 is attached to the base material BP, the rear surface BPb and the supporting area 15 - which will become support surfaces - are flattened and smoothed, and thus the biochip support member is completed.

As described above, this embodiment of the present invention makes the biochip easily measurable. Because the support area 15 is provided on the tip of the columnar member 10, there is no member for configuring the biochip support member CP on the periphery (the biomolecule forming side in the biochip BC (the back side of the side that is fixed to the support area 15)) of the biochip BC fixed to the support area 15. Therefore, measurement with a measuring device in a state of being as close as possible to the biochip BC becomes possible, and thus the biochip BC can be measured with high accuracy. Furthermore, because the columnar member 10 is attached to the base material BP through the attaching part JT in the present embodiment, adverse effects caused by deformation such as warping, which would occur when the base material BP and the columnar member 10 are integrally molded, can be suppressed. For example, when the base material BP and the columnar member 10 are integrally molded, some distortion such as warping would occur in the base material BP due to temperature distribution and release resistance. In this case, because the same height (the same Z position) is not achieved for the support areas 15 in the plurality of columnar members 10, focusing is necessary for each biochip BC when the measuring device measures the biochips BC fixed in the columnar members 10, and thus throughput would be reduced. By contrast, because the columnar member 10 is attached to the base material BP through the attaching part JT in the present embodiment, it is possible to suppress the variation in the heights of the support areas 15 that is caused by integral molding. Therefore, the time required for focusing during the measurement of the biochips BC fixed to the support areas 15 can be shortened, and a drop in throughput can be suppressed. The effects of focusing errors caused when focusing is performed a multiple of times can be eliminated. The biochips BC can be measured with high accuracy and high throughput. Furthermore, because the rear surface BPb of the base material BP and the support areas 15 are finished with a lapping treatment, and thus flattened and smoothed in the present embodiment, the heights (Z positions) of the support areas 15 can be made consistent with greater accuracy even when the thicknesses of the attaching parts JT do not become uniform during welding.

Furthermore, because the first hole part AL1 and the second hole part AL2 are provided in the base material BP in the present embodiment, the base material BP and the columnar member 10 can easily be positioned in, for example, a stage and the like by inserting a shaft member into both the first hold part AL1 and the second hole part AL2. Moreover, because the second hole part AL2 is an elongated hole that extends in the Y direction in the present embodiment, the base material BP can be positioned by fitting one of the shaft members in the elongated hole even when the distance between that axes of the shaft members has shifted from a predetermined value.

Furthermore, because the mark FM, which becomes an index for the position of the columnar member 10 in the columnar member 10 in the present embodiment, it is possible to easily confirm the position of the columnar member 10 by measuring the mark FM. In particular, because two marks FM are arranged parallel to the Y direction in the present embodiment, it becomes possible to, after one of the marks FM has been measured, measure the other mark FM by moving the base material BP only in the Y direction, and thus the time required to measure the marks FM can be shortened.

It should be noticed that the lapping treatment for the rear surface BPb of the base material BP described above, which was performed in the base material preparing step S2, may be executed in the lapping step S4 as well.

Furthermore, with regard to the base material BP, the front surface BPa may, in addition to the rear surface BPb, also be finished with a lapping treatment.

By this treatment, it is possible to flatten and smooth the surface to which the columnar member 10 will be mounted, and thus it is possible to suppress an increase in the amount of lapping treatment for the support area 15 that occurs due to the surface accuracy of the front surface BPa.

### [Method for Manufacturing the Biochip Support Member (Second Embodiment)]

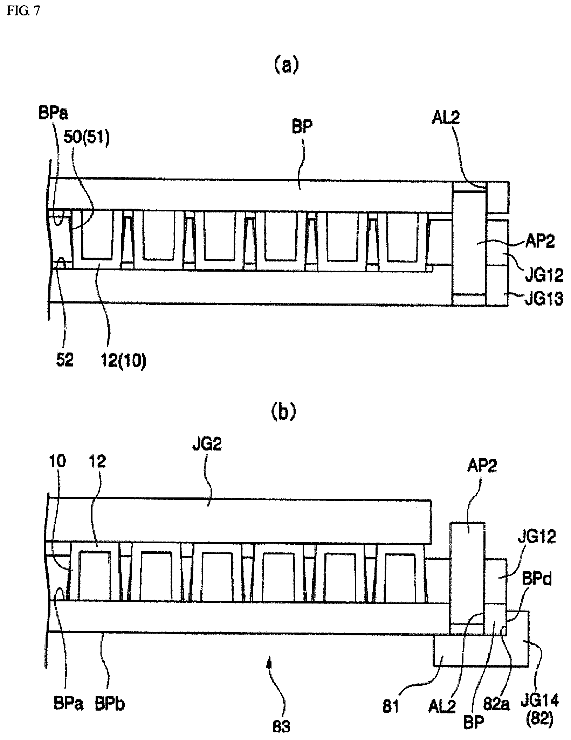

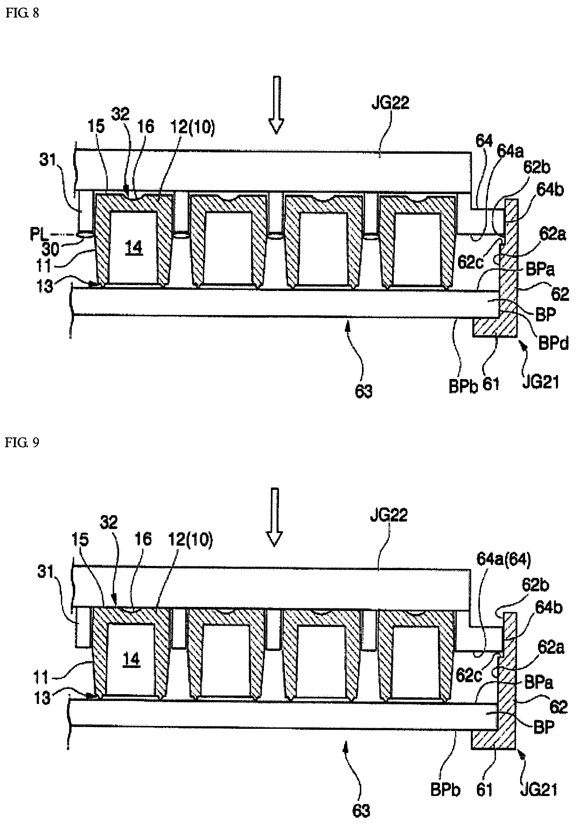

A second embodiment of the method for manufacturing the biochip support member will be described hereinafter with reference to FIG. 7 and FIG 8.

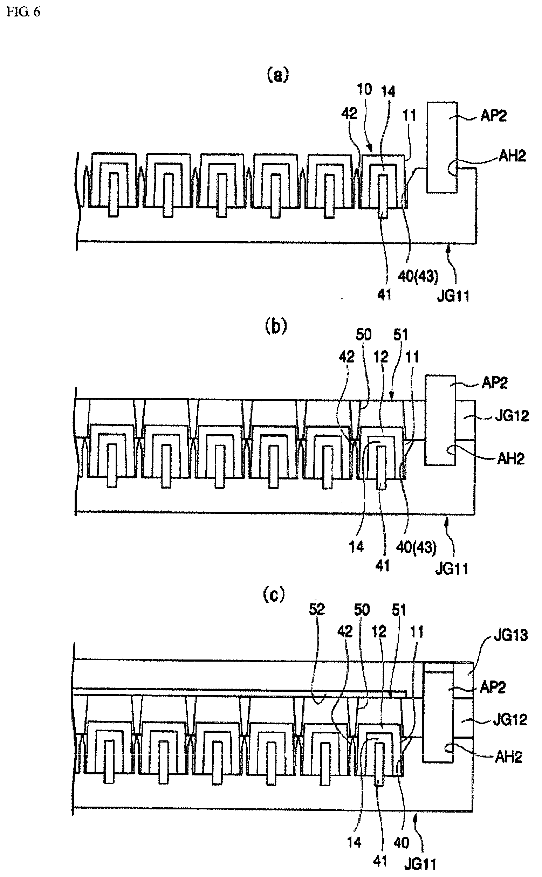

While an example illustrating a configuration where the columnar member 10 was mounted on the base material using a transport device that attaches and holds the member was described in the first embodiment of the present invention, a case will be described in the second embodiment of the present invention where a jig is used to align a plurality of the columnar members 10. FIG. 6 and FIG. 7 are cross sectional views. Note that, for convenience of discussion, the views illustrating the columnar member 10 and the protrusion 13 (FIG. 3) are omitted from FIG. 6 and FIG. 7.

The jig (first jig) JG11 illustrated in FIG 6 (a) provides a holding wall part (positioning part) 40 that holds the cylindrical part 11 of the columnar member 10 from an outer circumference side based on a position in which the columnar member 10 should be arranged in the base material BP, and a shaft 41 that is inserted in the internal space 14 of the columnar member 10 being held by the holding wall part 40. The holding wall part 40 forms a holding space 43 for holding the columnar member 10. An inclined surface 42 for guiding the columnar member 10 to the holding wall part 40 is provided on the tip part of the holding wall part 40. Furthermore, in the jig 11, a hole part AH1 with a bottom into which a positioning pin AP1 fits is provided in a position that corresponds to the first hole part AL1 of the base material BP, and a hole part AH2 with a bottom into which a second positioning pin AP2 fits is provided in a position that corresponds to the second hole part AL2 (FIG 6 illustrates only the positioning pin AP2 and the hole part AH2). The positioning pin AP1 is long enough to be able to fit into a jig JG12 and a jig JG13, which will be described later, in a state where the positioning pin AP1 is fitted in the hole part AH1. That is, the length of the positioning pin AP1 is set so that the amount protruding from the jig JG11 in a state where the positioning pin AP1 is fitted into the hole part AH1 is longer than the thickness of the jig JG12. The positioning pin AP2 is long enough to be able to fit into the jig JG12 and the jig JG13, which will be described later, in a state where the positioning pin AP2 is fitted in the hole part AH2. That is, the length of the positioning pin AP2 is set so that the amount protruding from the jig JG11 in a state where the positioning pin AP2 is fitted into the hole part AH2 is longer than the thickness of the jig JG12. Furthermore, the diameter of the positioning pin AP1 and the diameter of the positioning pin AP2 are formed to sizes that enable the positioning of the jig JG11, the jig JG12, the jig JG13 and the base material BP and allow the attachment and removal thereof.

A through hole 51 that is surrounded by a holding surface 50 that holds the outer circumference surface of the cylindrical part 11 is formed in a position facing the holding space 43 when the jig JG12 illustrated in FIG. 6 (b) is fitted on the positioning pin AP1 and the positioning pin AP2 and thus stacked on the jig JG 11. The size of the holding surface 50 (through hole 51) is set to a size so that the top panel 12 protrudes from the through hole 51 when the columnar member 10 (the outer circumference surface of the cylindrical part 11) is held from below.

A support surface 52 that supports the top panel 12, protruding from the jig JG12 from below, is provided when the jig JG13 illustrated in FIG 6 (c) is fitted on the positioning pin AP1 and the positioning pin AP2 and thus stacked on the small diameter side of the through hole 51 in the jig JG12.

The procedure for aligning the plurality of the columnar members 10 using the jig JG11, the jig JG12, and the jig JG13, and for attaching the plurality of columnar members 10 to the base material BP will be described.

First, the columnar member 10 is inserted into the holding space 43, as illustrated in FIG. 6 (a). At this time, the other end of the cylindrical part 11 is guided by the inclined surface 42 and the shaft 41 is inserted and guided into the internal space 14, and thus the columnar member 10 is smoothly inserted into the holding space 43 and held by the holding member 40.

After the plurality of columnar members 10 have been held in the holding space 43 of the jig JG11, the jig JG12 is stacked on the jig JG11 while being fitted onto the positioning pins AP1 and AP2, as illustrated in FIG. 6 (b). Next, the jig JG13 is stacked on the jig JG 12 while being fitted onto the positioning pins AP1 and AP2, as illustrated in FIG. 6 (c).

After the jigs JG12 and JG13 have been successively stacked, in that order, on the jig JG11 that holds the columnar member 10, these jigs JG11 through JG13 are turned upside down, and thus the top panels 12 of the plurality of columnar member 10 are supported all at once by the holding surface 52 of the jig JG13. Furthermore, as illustrated in FIG. 7 (a), the front surface BPa is caused to abut the other end of the columnar member 10 while being positioned by the positioning pins AP1 and AP2 being fitted into the first hole part AL1 and the second hole part AL2 of the base material BP. At this time, the outer circumference surfaces of the plurality of columnar members 10 are held in the holding surface 50 of the jig JG12 and thus abut with the positioned base material BP.

After the base material BP, the jig JG12 and the jig JG13 are reversed in position, the rear surface BPb of the base material BP is mounted on a holding tool JG14, as illustrated in FIG 7 (b). The holding tool JG14 provides a bottom wall 81 that supports the rear surface BPb of the base material BP from below, and a sidewall 82 that protrudes from an edge of the bottom wall 81. The bottom wall 81 has an opening 83. The opening 83 has a shape of, for example, rectangle. The opening 83 is at least as big as, for example, the area formed by the plurality of columnar members 10 in the base material BP. The bottom wall 81 supports the edge of the rear surface BPb of the base material BP from below. The bottom wall 81 supports an area that includes the outer edge (the four sides of the rear surface BPb of the base material BP) of the rear surface BPb of the base material BP. When supporting the base material BP, the bottom wall 81 does not make contact with an area (for example, a central area that includes the center of the rear surface BPb of the base material BP) other than the edge of the rear surface BPb of the base material BP. Because the bottom wall 81 has the opening 83, the contact surface area between the holding tool JG14 and the base material BP is small, and thus the absorption of the ultrasonic vibration generated by the ultrasonic horn can be suppressed. Because the bottom wall 81 has the opening 83, the base material BP and the holding tool JG14 can be prevented from being ultrasonically welded to together when the columnar member 10 is ultrasonically welded to the base material BP using the ultrasonic horn. The sidewall 82 protrudes from the edge of the bottom wall 81 toward the ultrasonic horn JG2 side. The sidewall 82 has a first side surface 82a that faces a side surface BPd of the base material BP. The first side surface 82a holds the side surface BPd of the base material BP supported by the bottom wall 81, and positions the base material BP within the XY plane.

After the jig JG13 is removed and the ultrasonic horn JG2 is caused to abut the support area 15, ultrasonic vibration and pressure are applied to the plurality of columnar members 10 using the ultrasonic horn JG2, as was described above, and thus the columnar member 10 is welded and fixed to the base material BP. Once the plurality of columnar members 10 are fixed to the base material BP, the biochip support member CP is completed by removing the holding tool JG1 and the ultrasonic horn JG2.

The subsequent lapping step is the same as for the first embodiment.

In this way, in addition to the fact that the similar operation and effect to the first embodiment can be obtained in the present embodiment, it is possible to easily arrange the plurality of columnar members 10 in predetermined positions on the base material BP through simple work using the jigs JG 11 through JG 14.

### [Method for Manufacturing the Biochip Support Member (Third Embodiment)]

The third embodiment of the method for manufacturing the biochip support member will be described hereinafter with reference to FIG. 8 and FIG 9.

In these figures, elements that are the same as the configuring elements of the first embodiment illustrated in FIG. 5 are given the same reference numerals and descriptions thereof are omitted. FIG. 8 and FIG. 9 are cross sectional views.

Examples of methods for manufacturing the columnar member 10 using, for example, pinpoint gate injection molding, were illustrated in the first and second embodiments, however, because these methods require the plurality of columnar members 10 to be released from a mold in a state of being separated from one another, the plurality of columnar members 10 must be aligned on the base material BP in subsequent steps. Therefore, a case where a side gate method is applied and a runner for injecting molten resin in the dent of a mold is used as a linking part and that thus links the plurality of columnar members 10 integrally, will be described in this embodiment.

Because the runner in the side gate method is normally formed facing a parting line, if the end surface of the cylindrical part 11 is made the parting line, as with the columnar member 10 illustrated in the first and second embodiments, there is a chance that the runner will also be welded because the welded part of the columnar member 10 and the runner are arranged close to one another. Therefore, in the present embodiment, the parting line is set in a position that is separated from the cylindrical part 11 in the axial direction of the columnar member 10, and a linking part that links the plurality of columnar members 10 is arranged in this position.

As illustrated in FIG. 8, a parting line PL is set for the columnar member 10 on a side close to the top panel 12 in the axial direction. The cylindrical part 11 reaches maximum diameter at the position of the parting line PL, and the outer circumference surface thereof is tapered so that the diameter reduces going away from the parting line PL. The other shapes for the columnar member 10 are the same as for the first embodiment of the present invention.

The plurality of columnar members 10 are linked by a linking part 30 that is formed by the runner so as to achieve a positional relationship arranged in the base material BP. The plurality of columnar members 10 and the linking part 30 are molded integrally by a single injection molding in the columnar member preparing step S1.

A holding tool JG21 and the ultrasonic horn JG2 used when the columnar member 10 is welded to the base material BP will be described next.

The holding tool JG21 provides a bottom wall 61 that supports the rear surface BPb of the base material BP from below, and an edge wall 62 that protrudes from an edge of the bottom wall 61. The bottom wall 61 has an opening 63. The opening 63 has a shape of, for example, rectangle. The opening 63 is at least as big as, for example, the area formed by the plurality of columnar members 10 in the base material BP. The bottom wall 61 supports the edge of the rear surface BPb of the base material BP from below. The bottom wall 61 supports an area that includes the outer edge (the four sides of the rear surface BPb of the base material BP) of the rear surface BPb of the base material BP. When supporting the base material, the bottom wall 61 does not make contact with an area (for example, an area of a central part that includes the center of the rear surface BPb of the base material BP) other than the edge of the rear surface BPb of the base material BP. Because the bottom wall 61 has the opening 63, the contact surface area between the holding tool JG21 and the base material BP is small, and thus the absorption of the ultrasonic vibration generated by the ultrasonic horn can be suppressed. Because the bottom wall 61 has the opening 63, the base material BP and the holding tool JG21 can be prevented from being ultrasonically welded together when the columnar member 10 is ultrasonically welded to the base material BP using the ultrasonic horn JG2. The sidewall 62 protrudes from the edge of the bottom wall 61 toward the ultrasonic horn JG2. The sidewall 62 provides a first side surface 62a that faces the side surface BPd of the base material BP on the bottom wall 61 side, a second side surface 62b provided more on the ultrasonic horn JG2 side and on the outside than the first side surface 62a, and a step 62c provided between the first side surface 62a and the second side surface 62b. The first side surface 62a holds the side surface BPd of the base material BP supported by the bottom wall 61, and positions the base material BP within the XY plane.

The ultrasonic horn JG2 provides a separating part 31 that positions the columnar members 10 in positions corresponding to each of the plurality of columnar members 10. The ultrasonic horn JG2 provides an engaging wall 64. The engaging wall 64 protrudes toward the base material BP in a position opposite the edge of the base material. The separating part 31 forms a holding space 32 for holding the outer circumference surface of each of the columnar members 10. The amount the separating member 31 protrudes is set, for example, to an amount that adds a margin (for example, about 0.5 mm) to the total of the distance relative to the axial direction (the vertical direction in FIG. 8) from the support area 15 to the linking part 30.

The engaging wall 64 provides an engaging surface 64a that faces the step 62c of the sidewall 62, and a holding surface 64b that is held by the second side surface 62b in a state of being positioned within the XY plane. The height position (Z position) of the engaging surface 64a is set so that the distance between the front surface BPa of the base material BP and the ultrasonic horn JG2 becomes the height of the columnar member 10 when the engaging surface 64a engages the step 62c of the sidewall 62.

The procedure for attaching the plurality of the columnar members 10 to the base material BP using the holding tool JG21 and an ultrasonic horn JG22 will be described.

First, the side surface BPd mounting the base material BP is held by the first side surface 62a of the sidewall 62 on the bottom wall 61 of the holding tool JG21, and thus the base material BP is positioned in the XY plane.

Next, the plurality of columnar members 10 linked by the linking part 30 are mounted on the side surface BPa of the base material BP. At this time, the plurality of columnar members 10 are linked so as to achieve a predetermined position by the linking part 30 and are thus mounted on the base material BP in a state where the relative positional relationship of the plurality of columnar members 10 is ensured. Furthermore, because the plurality of columnar members 10 are positioned using the separating part 31 in subsequent steps, it is not necessary to perform positioning with high accuracy when the plurality of columnar members 10 are mounted on the side surface BPa of the base material BP.

When the plurality of columnar members 10 linked by the linking part 30 are mounted on the base material BP, the ultrasonic horn JG22 is set on top of the columnar member 10 so that the separating part 31 faces the linking part 30, and the holding space 32 of each separating part 31 faces each columnar member 10. After this, the ultrasonic horn JG22 is lowered (brought closer to the columnar member 10). Here, before the holding surface 64b of the engaging wall 64 is held by the second side surface 62b of the sidewall 62, the upper parts only of the plurality of columnar members 10 are held by the holding spaces 32 of the plurality of separating parts 31. Furthermore, so that the holding surface 64b of the engaging wall 64 is held by the second side surface 62b of the sidewall 62 while the upper parts only of the plurality of columnar members 10 are being held by the holding spaces 32 of the plurality of separating spaces 31, the ultrasonic horn JG22 is moved down. By this, the ultrasonic horn JG22 is positioned within the XY plane and the plurality of columnar members 10 being held by the holding spaces 32 are positioned within the XY plane. After this, the ultrasonic horn JG22 is lowered (brought closer to the columnar member 10) so that the ultrasonic horn JG22 abuts with the top panels 12 of the plurality of columnar members 10. Here, because the separating part 31 protrudes from the ultrasonic horn JG22 by the protruding amount described above, the separating part 31 abuts with and is pushed into the linking part 30 before the ultrasonic horn JG22 makes contact with the top panels 12 of the plurality of columnar members 10, as illustrated in FIG 8, when the ultrasonic horn JG22 is moved in a direction so as to draw closer to the plurality of columnar members 10. As a result, shear force acts upon the linking part 30, and thus the linking part 30 is cut and separated from the plurality of columnar members 10, as illustrated in FIG 9.

After the separating part 31 separates the linking part 30 and the ultrasonic horn JG22 abuts with the top panels 12 of the plurality of columnar members 10, ultrasonic vibration and pressure are applied to the plurality of columnar members 10 using the ultrasonic horn JG22, in the same way as in the first embodiment, and thus the plurality of columnar members 10 are welded and fixed to the base material BP. Note that, despite the protrusion 13, there is a possibility that the height of the columnar member 10 will change because the end surface of the cylindrical part 11 melts to form the welded part (welded layer) when the ultrasonic vibration and pressure are applied to the plurality of columnar members 10. Because the engaging surface 64a of the engaging wall 64 engages the step 62c of the sidewall 62 in the present embodiment, the downward movement of the ultrasonic horn JG22 is regulated and thus the height of the columnar member 10 can be kept constant.

Once the columnar member 10 is fixed to the base material BP, the biochip support member CP is completed by removing the holding tool JG21 and the ultrasonic horn JG22.

The subsequent lapping step is similar to for the first embodiment.

In this way, because the same operation and effect as the first embodiment can be obtained and the plurality of columnar members 10 are linked and integrated by the linking part 30 in a predetermined positional relationship in the present embodiment, the plurality of columnar members 10 can easily be arranged in predetermined positions on the base material BP, and thus an improvement in manufacturing efficiency can be achieved. Furthermore, because the linking part 30 is separated during the movement for abutting the ultrasonic horn JG22 with the columnar member 10 in the present embodiment, there is no need to provide a separate step for separating the linking part 30, and thus a further increase in production efficiency can be achieved.

### [Biochip Package]

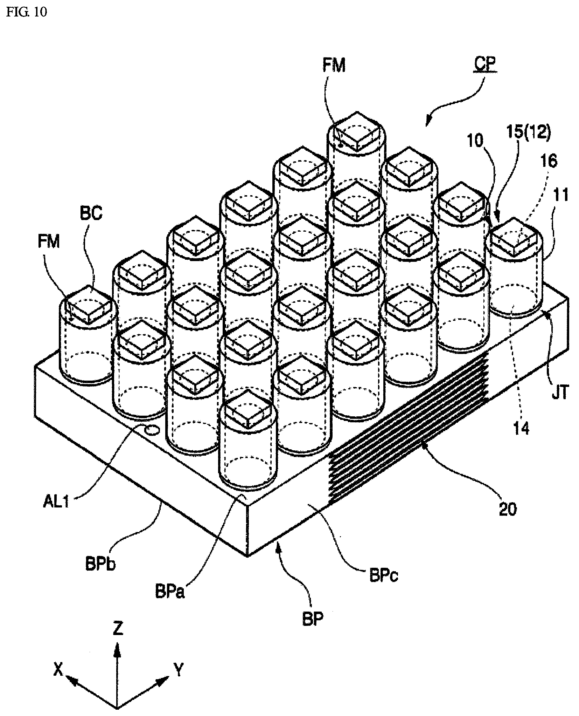

The biochip package (inspection package) that provides the biochip support member CP described above will be described hereinafter with reference to FIG 10 through FIG. 12.

In these figures, elements that are the same as the configuring elements illustrated in FIG. 5 are given the same reference numerals and descriptions thereof are omitted.

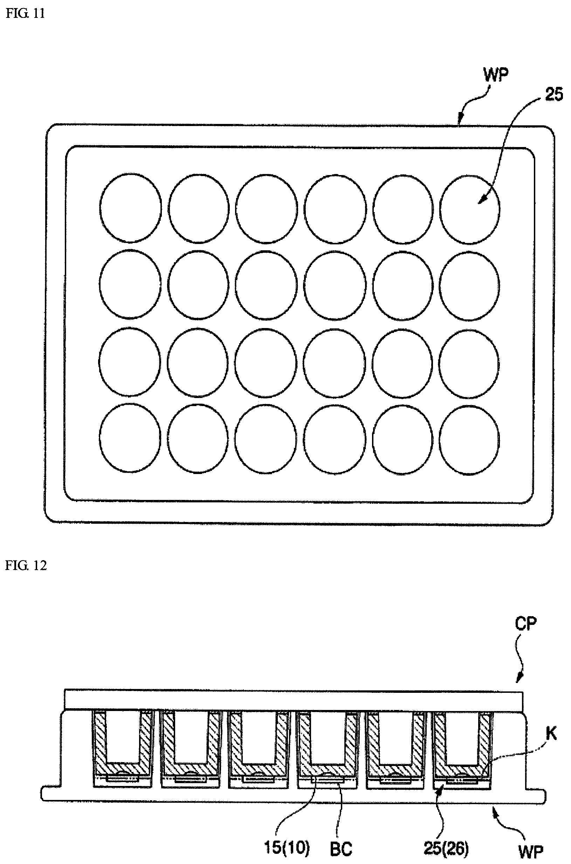

FIG 10 is an external perspective view of the biochip support member CP where the biochip BC is fixed to the support area 15. FIG 11 is a plan view of a well plate (holding member) WP that configures the biochip support member CP and a biochip package PG FIG. 12 is a cross sectional view of the biochip package PG.

The biochip BC has a substrate and a biomolecule. The substrate is, for example, a plate like member. The substrate has a first surface and a second surface that is on the opposite side from the first surface. The biomolecule is formed on the first surface side of the substrate. As illustrated in FIG. 10, the biochip BC is fixed and implemented in the support area 15 of the biochip support member CP described above in a biochip implementing step S12 that will be described later. The biochip BC is fixed in the support area 15 of the biochip support member CP on the second surface side of the substrate.

As illustrated in FIG. 11, the well plate WP provides a holding part 25 in a position that corresponds to each of the plurality of columnar members 10 in the biochip support member CP. As illustrated in FIG. 12, the holding part 25 provides a holding space 26 that holds a specimen K that contains a target that is able to react specifically with the biomolecule of the biochip BC. The depth of the holding space 26 is set to a value such that the biochip BC being supported by the support area 15 of the columnar member 10 does not make contact with a bottom even when the biochip support member CP is assembled with the well plate WP and the columnar member 10 is inserted into the holding space 26. The material that forms the well plate WP is not particularly limited, but it is preferable that the plate be formed using the same material as the biochip support member CP so that differences in the amounts of thermal expansion do not occur when the plate is integrated with the biochip support member CP.

The biochip package PG is a configuration that provides the well plate WP and the biochip support member CP described above.

### [Screening Device and Screening Method]

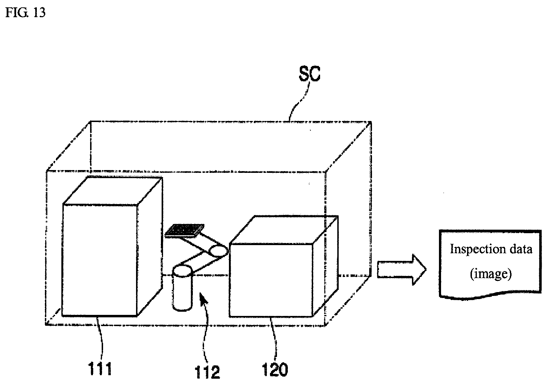

A device and method that perform screening using the biochip package PG described above will be described next with reference to FIG 13 through FIG 15.

FIG. 13 is a schematic view of a screening device SC. The screening device SC provides a dispenser 111, a measuring device 120, and a transport device 112. The screening device SC in the present embodiment provides a dispenser that dispenses the specimen K into the holding part 25 of the well plate WP in the biochip package PG described above, the measuring device 120 that detects the affinity between the biomolecule and the target contained in the specimen K, and the transport device 112 that transports the biochip (biomolecule array) BC from the dispenser 111 to the measuring device 120. For example, the dispenser 111 performs a dispensing process that dispenses the specimen that contains the target into the holding part 25 in the well plate WP. Furthermore, a reacting step, a cleaning and drying step, a package integrating step, and a package separating step, which will be described later, are all performed in the dispenser 111.

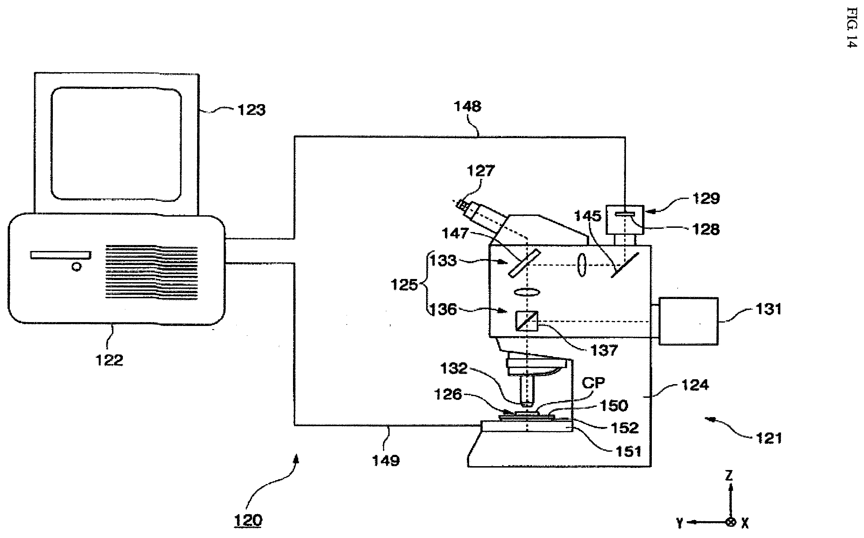

FIG. 14 is a schematic configuration diagram of the measuring device 120.

The measuring device 120 provides a measuring device main unit 121 for observing the biochip support member CP (the spot formed on the biochip BC), a controller 122 that controls the operation of the measuring device main unit 121, and a display 123 that is connected to the controller 122. The controller 122 includes a computer system. The display 123 includes a flat panel display such as, for example, a liquid crystal display.

The measuring device main unit 121 provides a light source 131, an optical system 125 that includes an object lens 132 and other optical elements, a stage (measuring stage) 126 that is movable while supporting the biochip support member CP, an eyepiece 127, and an observation camera 129 that includes a sensor 128 to receive light through an object. Examples of the sensor 128 may be photodetectors such as a photomultiplier tube PMT), a charge coupled device (CCD), and image elements such as a CMOS (here an image element is used as an example of the sensor 128 in the present embodiment). The measuring device main unit 121 provides a body 124 that supports light source 131, the optical system 125, the stage 126, the eyepiece 127 and the observation camera 129.

The optical system 125 provides an illuminating optical system 136 that illuminates the biochip support member CP using light irradiated from the light source 131, and an imaging optical system 133 that forms an image of the biochip support member CP being illuminated by the illuminating optical system 136 in the vicinity of the sensor 128 and the eyepiece 127. The sensor 128 and the eyepiece 127 are arranged on the imaging surface side of the imaging optical system 133.

The object lens 132 is an infinite system object lens that can face the biochip support member CP supported by the stage 126. In the present embodiment, the object lens 132 is arranged on the + Z side (upward) of the biochip support member.

The light source 131 is able to emit excitation light of a predetermined wavelength band for generating fluorescence from the biochip BC, and illuminating light in a predetermined wavelength band for observing the biochip.

The illuminating optical system 136 uses the light emitted from the light source 131 to illuminate the biochip BC using excitation light or illuminating light. The illuminating optical system 136 includes the object lens 132 and an optical unit 137 that is able to separate fluorescence from excitation light and illuminating light. The object lens 132 emits excitation light and illuminating light in order to illuminate the biochip BC. The illuminating optical system 136 illuminates the biochip BC being supported in the stage 126 using excitation light and illuminating light from above (the Z direction) as predetermined. Furthermore, the imaging optical system 136 transmits the fluorescence generated by the biochip BC and then guides the fluorescence to the imaging optical system 133.

The imaging optical system 133 includes an optical element 147 that separates out the light from the object lens 132 and a reflecting mirror 145, and forms an image of the biochip BC in the vicinity of the sensor 128 and the eyepiece 127. The optical element 147 includes a half mirror that transmits a portion of and reflects a portion of the light incident thereupon. The optical element 147 may be a dichroic mirror. Alternatively, the optical element 147 may be a total reflection mirror (for example, a quick return mirror) that has a function of switching a light path.

The stage 126 supports the biochip support member CP on the object surface side of the imaging optical system 133. The biochip support member CP is supported on the stage 126 so that the surface of the biochip BC being supported in the support area 15 faces the object lens 132.

A portion of the light from the biochip BC that incidents in the optical element 147 through the object lens 132, is transmitted through the optical element 147, is led to an eyepiece lens 143, and is emitted from the eyepiece 127. An image of the biochip BC (for example, a spot) is formed in the vicinity of the eyepiece 127 by the imaging optical system 133. By this image forming, an observer can confirm the image of the spot through the eyepiece 127.

Furthermore, a portion of the light from the biochip BC that incidents in the optical element 147 through the object lens 132 and an object lens 146, is reflected by the optical element 147 and the reflecting mirror 145, in this order, and then incidents on the sensor 128 of the observation camera 129. An image of the biochip BC (for example, a spot) is formed in the sensor 128 by the imaging optical system 133. By this, the sensor 128 of the observation camera 129 is able to capture the image information of the biochip BC (for example, a spot).

As illustrated in FIG 14, the sensor 128 of the observation camera 129 and the control device 122 are connected through a cable 148, and the image information (for example, an image signal) of the biochip BC (spot) captured by the sensor 128 is output to the controller 122 through the cable 148. The controller 122 displays the image information from the sensor 128 using the display 123. The display 123 can enlarge and then display the image information of the biochip BC (for example, a spot) captured by the sensor 128.

Furthermore, in the present embodiment, the stage 126 provides a stage surface plate 150, and a driving device 152 that moves the stage surface plate 150 above a base member 151. The stage surface plate 150 is able to move within the XY plane and in the Z direction above the base member 151. The stage 126 (drive device 152) and the controller 122 are connected by a cable 149, and the controller 122 is thus able to move the stage surface plate 150 that is supporting the biochip support member CP within the XY plane and in the Z direction using the driving device 152.

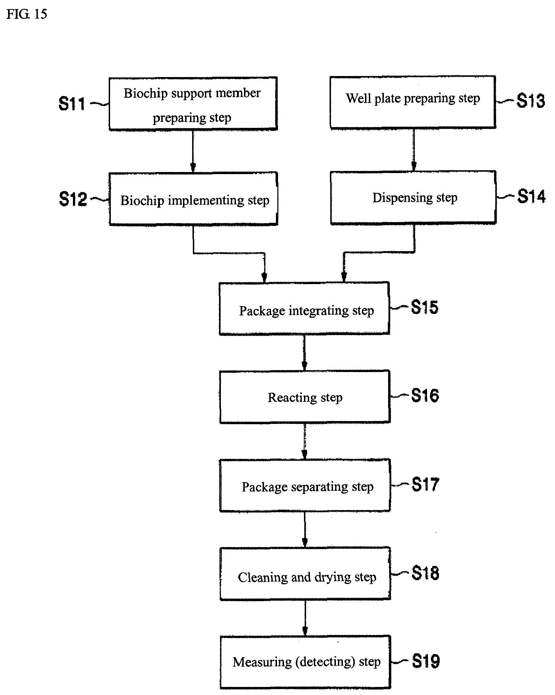

A method for screening the biochip BC (biomolecule array) using the biochip package PG will be described hereinafter using the flow chart illustrated in FIG 15.

The screening method in the present embodiment includes a biochip support member preparing step S11, a biochip implementing step S12, a well plate preparing step S 13, a dispensing step S14, a package integrating step S 15, a reacting step S16, a package separating step S 17, a cleaning and drying step S18, and a measuring (detecting) step S 19.

The biochip support member preparing step S11 includes the columnar member preparing step S1, the base material preparing step S2, the attaching step S3, and the lapping step S4 described above. The biochip implementing step S12 implements the biochip BC in the support area 15 of the biochip support member CP that was prepared in the preparing step S11. For example, an adhesive can be used as the method for fixing the biochip BC in the support area 15. Although thermosetting adhesive or a photocurable resin adhesive can be used as the adhesive, a photocurable resin adhesive is preferably used considering the effect of heat on the biomolecules. A configuration that applies the adhesive to the surface on the side opposite the side provided with the biomolecule of the biochip BC, a configuration that applies the adhesive to the support area 15 of the columnar member 10, or a configuration that applies the adhesive to both the surface on the opposite side and the support area 15 can be selected. Because the dent 16 is provided in the support area 15, excess adhesive is stored in the dent 16 during implementation of the biochip BC. Therefore, excess adhesive can be prevented from being squeezed out from the biochip BC and from the support area 15.

When the biochip BC is mounted in the support area 15, the biochip support member BC is first positioned by fitting shaft members in the first hole part AL1 and the second hole part AL2, and then the mark FM provided on the columnar member 10 is measured. By this, the position (center position) of the columnar member 10 on which the mark FM has been formed is confirmed. Because the position of the columnar member 10 and the position of another columnar member 10 relative to this position are well known, the position of the other columnar member 10 is derived relative to the position of the measured columnar member 10, and the biochip BC is thus implemented based on the derived position.

Because the mark FM is provided on two of the columnar members 10, the position of the other columnar member 10 may be corrected by deriving a scale error based on the results from measuring the two marks FM and the design positions of the mark FM, and then using the scale error. Here, because the X positions of the two marks FM are the same, both of the marks FM can thus be measured by moving the table that supports the biochip support member CP in the Y direction, and thus an increase in measuring efficiency can be achieved.

While the embodiment described above used a configuration that provided the mark FM on two of the columnar members 10, the mark may be provided on all of the columnar members 10. In this case, the implementation accuracy for the biochip BC can be raised because positions are detected for each of the columnar members 10. In this case as well, because the marks FM are arranged on a straight line that is parallel to the direction in which the columnar members 10 are aligned, a plurality of marks FM can be measured easily by moving the table in one direction when the marks FM are measured.

In the well plate preparing step S13, the well plate WP described above is manufactured by, for example, injection molding and then prepared. In the dispensing step S 14, the specimen K containing the target that is able to react specifically to the biomolecule provided in the biochips BC is dispensed, relative to the prepared well plate WP, into each of the holding parts 25 by the dispenser 111. The dispensed amount of the specimen K is set to an amount such that the biochip BC is soaked therein when the columnar member 10 is inserted into the holding part 25.

Dispensing the specimen K is performed using, for example, a nozzle. The dispensing nozzle can employ a procedure where a single nozzle dispenses the specimen in order into a plurality of holding parts 25, a procedure where a plurality of the dispensing nozzles are moved integrally all at once relative to the well plate WP, or a procedure where a plurality of the dispensing nozzles are moved independently relative to the well plate WP.

After the specimen K is dispensed into the holding part 25, the columnar member 10 is inserted into the holding part 25 (holding space 26) in the package integrating step S15 so that the biochip BC becomes soaked with the specimen K, as illustrated in FIG. 12, and thus the well plate WP and the biochip support member CP are integrated as the biochip package PG.

In the reacting step S16, the biochip BC (biomolecule) performs a reaction process between the biomolecule soaked in the specimen K and the target contained in the specimen K for a predetermined period of time. In order to promote the reaction in this reaction process, it is preferable to apply, for example, vibration or rocking to the biochip package PG to stir the specimen K.

After the reacting step has been performed for a the predetermined amount of time, the well plate WP and the biochip support member CP are separated in the package separating step S 17. In the cleaning and drying step S18, a cleaning process and a drying process are performed relative to the biochip BC of the biochip support member CP from which the well plate WP was separated in the package separating step S 17. In the cleaning process, for example, a cleaning solution is supplied to the biochip support member CP and the biochip BC is cleaned. When the cleaning process relative to the biochip BC is complete, the drying process is performed. In the drying process, the cleaning solution adhering to the biochip BC is dried using a blower and the like.

After the cleaning and drying step S18 is finished, the process proceeds to the measuring (detecting) step S 19. The well plate WP from which the biochip support member CP has been removed is transported by the transport device 112 and mounted on the stage 126 of the measuring device 120, and then the measuring process is performed.

During transport of the biochip support member CP, the transport device 112 grasps and grips the gripping part 20, however, because the grooves 21 that extend in the Y direction are formed with V shaped cross sections in the gripping part 20, the biochip support member CP can be transported in a stable manner during transport without causing problems such as tilting.

In the measuring (detecting) step S19, the affinity between the target contained in the specimen K and the biomolecule of the biochip BC, in the spot (for example, the biomolecule) formed in the biochip BC, is detected by the measuring device 120 described above. For example, when the target is fluorescently labeled by a fluorochrome, the measuring device 120 detects the affinity based on the intensity of the fluorescence of the fluorochrome generated by the excitation light.

For example, after the surface of a predetermined biochip BC is positioned in a predetermined position in the Z direction, the biochip support member CP is moved within the XY plane to a first imaging area where a predetermined (predetermined number of) spot in the biochip BC can be measured, and the image of the spot is imaged using the illuminating light for observation.

Next, the measuring device 120 selects and emits illuminating light from the light source 131 to illuminate the surface of the biochip BC. The illuminating light emitted from the light source 131 is separated into reflected light and transmitted light by the optical unit 137, is partially reflected and partially transmitted, and the partially reflected illuminating light is transmitted through the object lens 132, and then illuminates the surface of the biochip BC. The illuminating light reflected by the surface of the biochip BC is transmitted through the object lens 132 and the optical unit 137, and then incidents in the optical element 147.

Furthermore, a portion of the illuminating light that incidents in the optical element 147 is transmitted through the optical element 147, is led to an eyepiece lens 127, and is emitted from the eyepiece 127. By this, an image of the biochip BC (for example, a spot) is formed in the vicinity of the eyepiece 127. Furthermore, a portion of the illuminating light that incidents in the optical element 147 is reflected by the optical element 147 and the reflecting mirror 145, in that order, and then incidents in the sensor 128 of the observation camera 129.

By this, the image of a plurality of spots within a field of view sized based on the imaging properties of the sensor 128 and a predetermined magnification is formed in the sensor 128. The sensor 128 captures the image information (received the light information for the spots) and the image information (position information) of alignment marks provided on the biochip BC, which are not illustrated in the figure. The controller 122 stores the image information for the spots and derives, and also stores the arrangement (X, Y, θZ) of the group of spots in the field of view based on the position information of the alignment marks.

After this, the measuring device 120 switches the light being emitted from the light source 131 to, for example, excitation light of a predetermined wavelength band in order to perform the fluorescence measurement. The illuminating light emitted from the light source 131 is reflected (total reflection) by the optical unit 137, is transmitted through the object lens 132, and then illuminates the surface (for example, spots) of the biochip BC. Fluorescence is generated from the spots, among the plurality of spots illuminated by the excitation light, that are bonded by the specific reaction between the target contained in the specimen and the biomolecule. The generated fluorescence is transmitted through the object lens 132 and the optical unit 137, and then incidents in the optical element 147. For example, when the affinity of the spots bonded by the specific reaction is high affinity, the detected fluorescence intensity is high.

Furthermore, just as with the measurement of the spots using illuminating light, the image of spots generating fluorescence is formed in the vicinity of the eyepiece 127 and, at the same time, formed within the field of view of the sensor 128. The sensor 128 captures the image information (received light information for the spots) for the spots that generated fluorescence.

In the spot measurement using the illuminating light and the spot measurement using the excitation light, the biochip BC locates at the highest (that is, closest to the object lens 132) position in the biochip support member CP in the optical axis direction or in a direction parallel to the optical axis of the object lens 132. After the measurement of the first image area in the biochip BC is complete, the measuring device 120 moves the biochip support member CP so that the biochip BC is positioned in a second imaging area adjacent to the first imaging area. The second imaging area is set in a position where a portion of the alignment marks imaged in the first image area can be imaged in the field of view of the sensor 128. Furthermore, in the same way as for the imaging relative to the first image area, the measuring device 120 implements the measurement of the spots and the alignment marks using the illuminating light and the measurement of spots using fluorescence.

Furthermore, after implementing the measurement process for a plurality of imaging regions until the measurement of all of the spots is completed, the controller 122 uses the measurement results of the alignment marks from each of the imaging regions to perform screen synthesis of the results from the spot measurements that uses the illuminating light. The addresses in the biochip BC of the spots, bonded by the specific reaction between the target of the specimen K and a biomolecule 72, can be measured by comparing the screen synthesized measurement results.

With the present embodiment, it is possible to measure a biochip simply and easily. Deformation such as warping and the like of the biochip support member CP is suppressed, furthermore, because the rear surface BPb of the base material BP in the biochip support member CP, which is the surface supported by the stage, and the biochip are supported and because the support areas 15 provided by the plurality of columnar members 10 are flattened and smoothed, focus adjustment during measurement of the biochips supported in the support areas 15 can be minimized. Therefore, it is possible to prevent a drop in throughput when the biochip BC is measured.

The preferred embodiments of the present invention were described above referring to the attached figures, however, needless to say that the present invention is not limited to these examples. The forms and combinations of the configuring members illustrated in the examples described above are merely examples, and thus a variety of modifications are possible based on design requirements within the scope of the present invention.

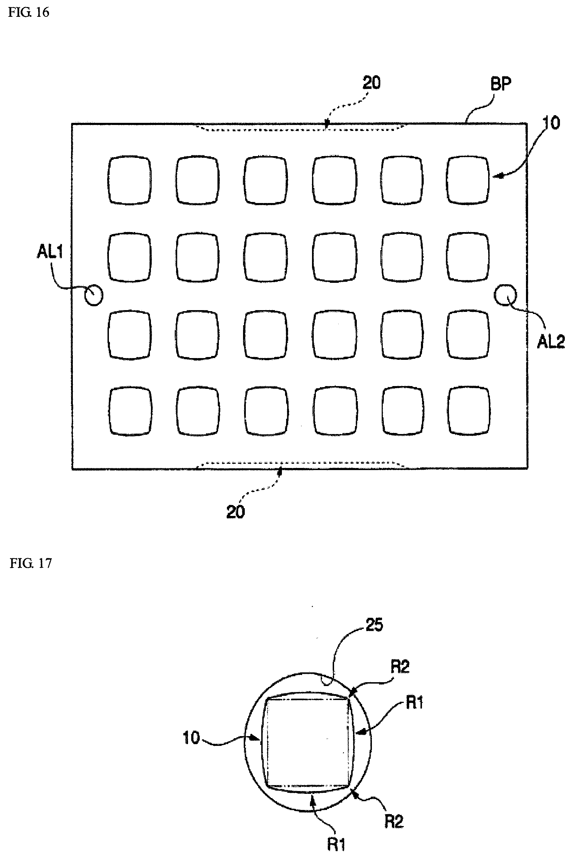

For example, in the embodiments described above, examples of configurations were illustrated where both the columnar member 10 of the biochip support member CP and the holding part 25 of the well plate WP were round when viewed in a plane, and the gap amount between the holding part 25 and the columnar member 10 was constant, however, the amount is not limited to this and thus a configuration may be used that provides areas where the gap amounts vary. For example, as illustrated in FIG. 16 and FIG. 17, the columnar member 10 may be formed into a shape where R chamfering is applied to corners relative to a quadrangular prism and the four side surfaces are formed as curved surfaces. As an example, the columnar member 10 may have a shape where four side surfaces are formed as curved surfaces (first area) R1, and the portions of the corners where these curved surfaces R1 intersect are given the roundness (so-called, rounded chamfer) of a curved surface (second area) R2 that has a curvature that is larger than the curved surface R1. By employing this configuration, the gap amount between the curved surface R1 and the holding part 25 and the gap amount between the curved surface R2 and the holding part 25 can be made to vary.

When the gap between the columnar member 10 and the holding part 25 is constant and the specimen K is placed into the gap, the holding space 26 can become negatively pressurized when the columnar member 10 is removed from the holding part 25, making the columnar member 10 difficult to remove and creating a concern that package separation will be hindered. Therefore, as illustrated in FIG 16 and FIG. 17, by varying the gap amounts between the columnar member 10 and the holding part 25, air readily enters from areas where the gap is large, and thus package separation becomes easy.

Furthermore, examples of configurations were illustrated in the embodiments described above where the attaching part JT that attaches the columnar member 10 to the base material BP is a welded part, however, the configurations are not limited to this and thus, for example, the configuration may be one where the columnar member 10 is attached to the base material BP using an adhesive. While the adhesive used in this case may be either a thermosetting adhesive or a photocurable resin adhesive, a photocurable resin adhesive is preferably used considering the effect of the heat that is applied to the base material BP.

A columnar member 10 having an internal space 14 was used in the embodiments described above, however, the member is not limited to this and thus a solid columnar member may be used, and a rod-like member may be used. Note that the shape of the columnar member 10 may be a round cylinder, a square cylinder, or a convex shape. A columnar member of a convex shape, as an example, may be one where the bottom surface and the top surface are round and the surface area of the top surface is smaller than the surface area of the bottom surface, or may be another where the bottom surface and the top surface are rectangular and the surface area of the top surface is smaller than the surface are of the bottom surface. Furthermore, even when the columnar member 10 is made of a resin material, the manufacturing method therefor is not limited to injection molding and thus other methods such as extrusion molding may be used. Moreover, the materials for the columnar member 10 and the base material BP are limited to the resin material described above. For example, the configuration may be one where the columnar member is formed using a fused quartz rod and the base material BP is formed using borate silicate glass, and thus a configuration where the two are attached through an adhesive.

The base material BP may have a marked part on the first side surface BPa thereof for the positioning of an area or attaching the columnar member 10 by welding. In a case where the columnar member 10 is attached to the first surface BPa of the base material BP by welding, the marked part indicates the position to which the columnar member 10 will be attached. The marked part may be, for example, a linear mark or a groove into which the columnar member 10 is inserted.

The embodiment described above illustrated examples of configurations where 6 x 4, or 24, of the columnar members were aligned in the base material, however, this is an example, and thus it goes without saying that the configuration may be one where a single columnar member 10 is attached to the base material BP or may be a configuration that aligns a different number (for example, 12x8, or 96) columnar members.

### (Reference Sings List)

10...columnar member, 11...cylindrical part, 12...top panel, 13...protrusion, 14...internal space, 15...support area, 16...dent, 17...opening, 20...attaching part, 25...holding part, 30...linking part, 31...separating part, 40...holding wall part (positioning part), 111...dispensing device, 120...measuring device (detector), BC...biochip, BP...base material, BPa...front surface (first surface), BPb...rear surface (second surface), BPc...side surface (third surface), CP...biochip support member, FM...mark, JG2...ultrasonic horn (oscillator), JG11...jig (first jig), JG12...jig (second jig), JT...attaching part (welded part), K...specimen, PG...biochip package, SC...screening device, WP...well plate (holding member)

## Claims

1. A biochip support member, comprising:
a base material; and
a columnar member that is different from the base material, having a support area that can support a biochip formed of a biomolecule provided on one end, and is attached to the base material through an attaching part at the other end.

2. The biochip support member according to Claim 1, wherein the attaching part is formed in at least one of the base material or the columnar member, and the columnar member is attached to the base material by melting the attaching part by welding.

3. The biochip support member according to Claim 1 or 2, wherein the columnar member has an opening on the other end and forms a closed space with the base material.

4. The biochip support member according to any one of Claims 1 through 3, wherein the columnar member provides a dent that is formed in the support area.

5. The biochip support member according to any one of Claims 1 through 4, wherein the columnar member provides a mark that becomes an index for the position of the columnar member.

6. The biochip support member according to Claim 5, wherein the mark is provided in the support area.

7. The biochip support member according to Claim 6, wherein a plurality of the columnar members are attached to the base material, and the mark is provided in the support area of each of the plurality of columnar members.

8. The biochip support member according to Claim 7, wherein at least a portion of the plurality of columnar members are aligned with the centers of the support areas along a predetermined direction, and the mark is provided on a straight line that is parallel to the predetermined direction.

9. The biochip support member according to c Claim 8, wherein the mark is provided on a straight line that passes through the center of the support area of each of the plurality of columnar members.

10. The biochip support member according to any one of Claims 7 through 9, wherein the base material includes a first surface to which the columnar member is attached through the attaching part, and a second surface on the opposite side from the first surface, and provides a hole that passes through from the first surface to the second surface and an elongated hole that passes through from the first surface to the second surface.

11. The biochip support member according to Claim 10, wherein the hole and the elongated hole are provided on the base material so that a straight line that passes through the center of the hole and the center of the elongated hole is parallel to the straight line that passes through the center of the support area of each of the plurality of columnar members.

12. The biochip support member according to Claim 10 or 11, wherein the base material includes a third surface that is formed along the outer edge of the first surface and the outer edge of the second surface, and provides a groove that is provided in the third surface.

13. A biochip support member, comprising:
a base material; and
a columnar member that is a different member than the base material and has a support area that can support a biochip formed of biomolecules provided on one end, and a welded part interposed between the base material and the other end of the columnar member.

14. The biochip support member according to Claim 13, wherein the welded part is formed by curing a portion of at least one of the base material or the columnar member after melting.

15. A biochip package, comprising:
the biochip support member according to any one of Claims 1 through 14; and
a holding member that has a holding part that is able to hold a specimen that contains a target that is able to react specifically to a biomolecule.

16. The biochip package according to Claim 15, wherein the holding part and the columnar member form a first area of a first gap amount and a second area of a second gap amount when the columnar member is inserted into the holding part.

17. A screening device, comprising:
a dispenser for dispensing a specimen into the holding part in the biochip package according to Claim 15 or 16; and
a detector for detecting the affinity between a target contained in the specimen and a biomolecule.

18. A method for manufacturing a biochip support member, comprising:
a first step that prepares a base material;
a second step that prepares a columnar member that is a different member than the base material and has a support area that can support a biochip formed of a biomolecule provided on one end; and
a third step that is attaches the other end of the columnar member to the base material through an attaching part.

19. The method for manufacturing a biochip support member according to Claim 18, wherein the third step includes a step that melts a portion of at least one of the base material or the columnar member as the attaching part, and a step that welds the other end of the columnar member to the base material.

20. The method for manufacturing a biochip support member according to Claim 18 or 19, wherein the third step includes a step that applies ultrasonic vibration and pressure to at least one of the base material or the columnar member.

21. The method for manufacturing a biochip support member according to any one of Claims 18 through 20, further comprising:
a fourth step that provides a lapping treatment to the support area.

22. The method for manufacturing a biochip support member according to Claim 21, wherein the lapping treatment is performed after the third step.

23. The method for manufacturing a biochip support member according to Claim 21 or 22, wherein the base material provides a first surface to which the other end of the columnar member is attached and a second surface on the side opposite from the first surface, further comprising:
a step that provides a lapping treatment to the second surface of the base material prior to the fourth step.

24. The method for manufacturing a biochip support member according to Claim 23, further comprising:
a step that provides a lapping treatment to the first surface of the base material prior to the third step.

25. The method for manufacturing a biochip support member according to any one of Claims 18 through 24, wherein the second step includes a step that integrally forms a plurality of the columnar members with a linking part that links the plurality of columnar members in an arrangement where each is attached to the base material.

26. The method for manufacturing a biochip support member according to Claim 25, wherein the linking part links the columnar member in a position separated from the tip of the other end in the axial direction of the columnar member.

27. The method for manufacturing a biochip support member according to Claim 25 or 26, further comprising:
mounting the plurality of the columnar members linked by the linking part in the base material;
moving the columnar member and an oscillator relative to the axial direction of the columnar member in order to abut with the oscillator that melts a portion of at least one of the base material or the columnar member; and
separating the columnar member and the linking part using a separating part provided in the oscillator during the movement relative to the columnar member and the oscillator, prior to the third step.

28. The method for manufacturing a biochip support member according to any one of Claims 18 through 24, further comprising:
setting and positioning the plurality of columnar members in a jig having a positioning part for positioning the plurality of columnar members in a position where each is attached to the base material; and
abutting the base material with the plurality of columnar members positioned using the jig, prior to the third step.

29. The method for manufacturing a biochip support member according to Claim 28, further comprising:
positioning the plurality of columnar members using the first jig that supports the other end of the plurality of columnar members from below and performs the positioning;
positioning and attaching a second jig that supports the other end of the plurality of columnar members from below and performs positioning to the first jig that supports the plurality of columnar members from below;
turning the positioned first jig and second jig upside down and then supporting and positioning one end of the plurality of columnar members using the second jig;
removing the first jig from the second jig; and
abutting the base material with the end of the plurality of columnar members one end of which is supported from below.

30. A screening method, comprising:
preparing the biochip support member manufactured by the method for manufacturing a biochip support member according to any one of Claims 18 through 29;
preparing a holding part that is able to hold a specimen containing a target that is able to react specifically to a biomolecule;
arranging and fixing the biochip in the support area of the columnar member;
dispensing the specimen in the holding part so that the biochip is soaked in the specimen;
causing a reaction between the target and the biomolecule in the holding part into which the specimen was dispensed; separating the biochip support member and holding member after the reaction; and
detecting the affinity between the target and the biomolecule.

31. The screening method according to Claim 30, further comprising:
detecting a mark that becomes an index of the position of the provided columnar member in the columnar member prior to arranging the biochip in the support area.
